# EUROPEAN PATENT SPECIFICATION

(11) **EP 2 895 079 B1**
(45) Date of publication and mention of the grant of the patent: **21.06.2017**
(21) Application number: 13854303.8
(22) Date of filing: 24.10.2013
(51) Int. Cl.: A61B 17/00, A61B 17/29, A61B 17/30, A61F 9/007

(54) **DISPOSABLE CAPSULORHEXIS FORCEPS**
EINWEG-KAPSULORHEXISPINZETTE
PINCETTE JETABLE POUR CAPSULORHEXIS

(30) Priority: 13.11.2012 US 201213675509
(43) Date of publication of application: 22.07.2015
(73) Proprietor: Alcon Research, Ltd., Fort Worth, Texas 76134 (US)
(72) Inventor: SCHALLER, Philipp, CH-8260 Stein am Rhein (CH); GRUEEBLER, Reto, CH-8606 Greifensee (CH)
(74) Representative: Hanratty, Catherine
(86) International application number: PCT/US2013/066575
(87) International publication number: WO 2014/078049

(56) References cited:
- WO-A1-97/15234
- US-A- 5 752 960
- US-A1- 2001 056 286
- US-A1- 2008 064 929
- US-A1- 2009 012 519
- US-A1- 2010 168 787
- US-B2- 7 758 274
- US-B2- 8 235 978

## Description

### TECHNICAL FIELD

The present disclosure generally relates to surgical instruments, and in particular to a capsulorhexis forceps for use in ophthalmic surgeries, that can have a disposable forceps tip that can be inexpensively molded or extruded.

### BACKGROUND

Cataracts occur when the natural lens of a person's eye or its surrounding transparent membrane becomes clouded, resulting in various degrees of visual impairment. In response to the development of cataracts in a person's eye, several surgical techniques have been developed for cataract extraction. In general, during such cataract surgeries, the surgeon will make an incision through an anterior portion of the lens capsule of the eye to create a flap or opening through which the surgeon can remove the damaged portion of the eye. For example, in a continuous curvilinear capsulorhexis type of capsulotomy, the periphery of the lens generally is opened using the sharp tips of a capsulorhexis forceps. The tips of the forceps are then used to create an opening through which damaged lens material can be removed and an artificial replacement lens can be inserted.

The principal tool used for such cataract surgeries typically is a capsulorhexis forceps. Such capsulorhexis forceps generally are formed with a handle portion designed to be grasped by the surgeon and a pair of sharp tips that are used for both creating a flap and tearing the tissue, and for grasping the lens.

Document WO97/15234 discloses a forceps device according to the preamble of claim 1.

### SUMMARY

The forceps device of the invention is defined in independent claim 1. Preferred embodiments are defined in the dependent claims.

According to one aspect, the disclosure describes a forceps device including a handle, a forceps tip, and a sleeve. The handle may include a first arm element, a second arm element, the first arm element and the second arm element joined at a first end of the handle, an elongate body extending between the first arm element and the second arm element, and a locking mechanism formed in the elongate body. The forceps tip may be coupled to the handle and may include a pair of pincers extending from a distal end of the forceps tip. The pair of pincers may be laterally offset from each other to define an open configuration. The pincers may be moveable between the open configuration and a closed configuration in which the pair of pincers contact each other. The forceps tip may also include a tip connector extending from a proximal end of the forceps tip. The tip connector may be adapted to interlock with the locking mechanism. The sleeve may be coupled to the arm elements and define a central passage. The forceps tip may extend through the central passage. The sleeve may be operable to move relative to the forceps tip and actuate the pincers between the open configuration and the closed configuration in response to lateral displacement of the arm elements.

Another aspect of the disclosure encompasses a forceps that includes a forceps tip having a first end and a second end, a pair of resilient pincers formed at the first end of the tip body and moveable between an open position and a closed position, and a tip connector formed at the second end of the forceps tip. The tip connector may include a plurality of teeth adapted to selectively engage a handle. The pincers may be biased toward the opened position.

The various aspects may include one or more of the following features. Resilient elements may extend between distal ends of the arm elements and the sleeve. A distal end of at least one of the pincers may include a grasping surface. The grasping surface may include a series of serrations. The forceps tip may include a tip body and a tip extension coupled to the tip body. The tip body may include the pair of pincers, and the tip extension may include the tip connector. The tip connector may include a first plurality of teeth arranged longitudinally along the forceps tip. The locking mechanism may include a, second plurality of teeth. The second plurality of teeth may be configured to interlock with the first plurality of teeth to couple the forceps tip to the handle. The first plurality of teeth may include a first set of teeth extending longitudinally along a first side of the tip connector and a second set of teeth may extending longitudinally along a second side of the tip connector. The second plurality of teeth may include a third set of teeth configured to interlock with the first set of teeth and a fourth set of teeth configured to interlock with the second set of teeth. The first set of teeth and the second set of teeth may be longitudinally offset from each other. The third set of teeth and the fourth set of teeth may be offset from each other by the same amount the first set of teeth is offset from the second set of teeth.

The forceps tip may be formed from a plastic injection molded material. The forceps tip may be formed from a metal injection molded material. The locking mechanism may also include a first locking member and a second locking member. The first set of teeth may be formed on the first locking member, and the second set of teeth may be formed on the second locking member. The first locking member and the second locking member may be laterally movable. The handle may also include a first resilient element extending between the first arm element and the sleeve and a second resilient element extending between the second arm element and the sleeve.

The various aspects may also include one or more of the following features. The first end of the forceps tip may also include grasping surfaces formed on facing surfaces of the pincers. The grasping surfaces may include serrations. The plurality of teeth may include a first set of teeth arranged in series along a first side surface of the tip body and a second set of teeth arranged in series along a second side surface of the tip body. The first set of teeth may be offset from the second set of teeth. Each of the teeth of the first set of teeth and the second sets of teeth may have a tooth length, and the first set of teeth may be offset from the second set of teeth by a distance of approximately one half of the tooth length. The tip body may be formed from a material selected from the group comprising an injection molded plastic, a composite material, and a metal injection molded material.

Various features, objects and advantages of the present will become apparent to those skilled in the art upon a review of the following detailed description, when taken in conjunction with the accompanying drawings.

### DESCRIPTION OF THE DRAWINGS

FIG. 1 is a perspective view of an example capsulorhexis forceps tip.
FIG. 2A is a perspective view of another example capsulorhexis forceps tip.
FIG. 2B is a perspective view of an example capsulorhexis forceps tip with an extension member attached thereto.
FIG. 2C is a side view of an example capsulorhexis forceps tip with a movable sleeve extending along a tip body.
FIG. 3 is a perspective view of an example tip connector.
FIGs. 4A-4C are perspective views of example capsulorhexis forceps tips.
FIG. 5 is a perspective view of an example capsulorhexis forceps mounted within a handle assembly with an insert connector.
FIG. 6 is a side view of an example capsulorhexis forceps tip mounted within a handle assembly with an insert connector.
FIG. 7 is a detail view of an example forceps tip in which the grasping surfaces of the pincers are angled.
FIG. 8 is a perspective view of an example locking mechanism of the insert connector of the handle of FIG. 6 engaging a tip connector of the forceps tip.
FIG. 9 is a cross-sectional view of a locking mechanism of the example device of FIG. 6.
FIG. 10 is an end view of a sleeve adapted to define a fully actuated position of the example device of FIG. 6.
FIG. 11 is a detail view of grasping surfaces of an example capsulorhexis forceps tip.
FIGs. 12 and 13 show alternative locking mechanisms operable to adjustably secure a forceps tip.

Those skilled in the art will appreciate and understand that, according to common practice, the various features of the drawings discussed below are not necessarily drawn to scale, and that the dimensions of various features and elements of the drawings may be expanded or reduced to more clearly illustrate the various implementations described herein.

### DETAILED DESCRIPTION

Referring now to the drawings in greater detail in which like numerals indicate like parts throughout the several views, capsulorhexis forceps adapted for use in ophthalmic surgeries such as for the correction of cataracts in a patient's eye are described.

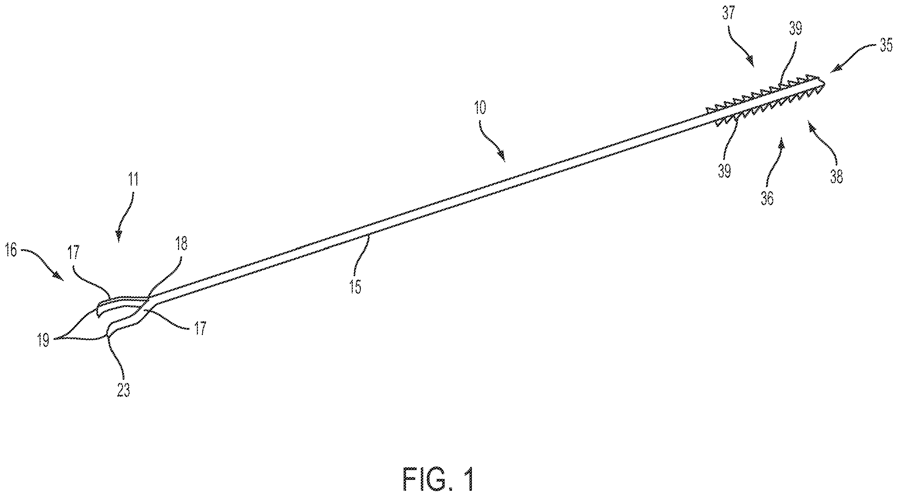

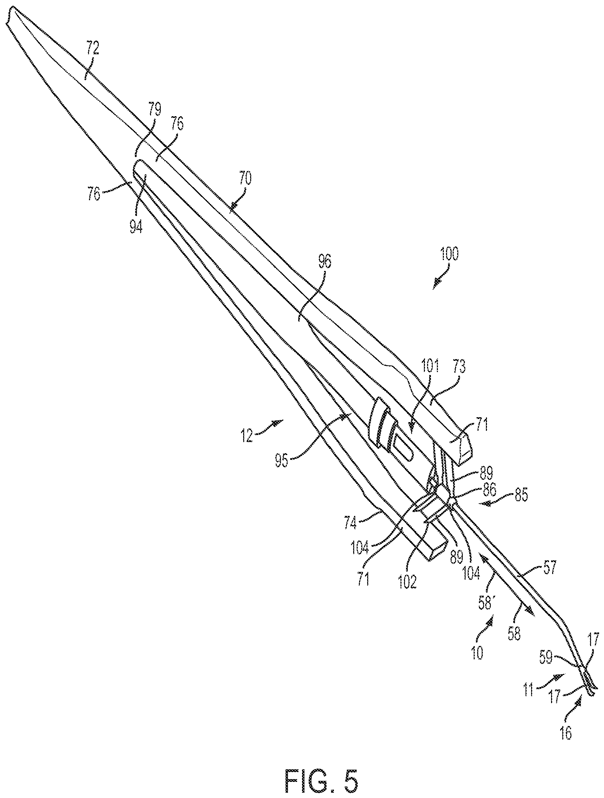

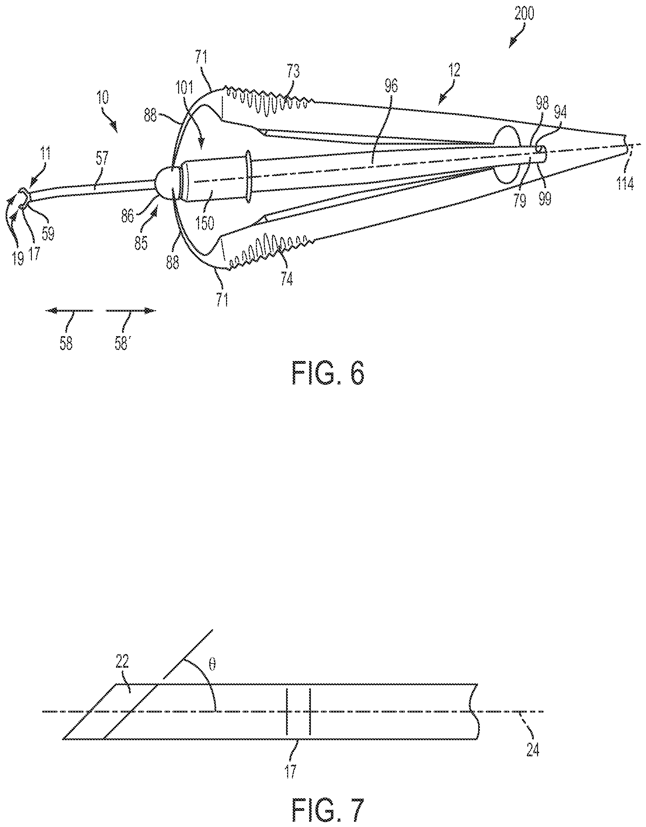

FIGs. 1-4C illustrate various example capsulorhexis forceps 10 and a forceps tip 11 thereof. FIGS. 5 and 6 further illustrate example implementations of the capsulorhexis forceps 10 included with different handle assemblies 12 (FIGs. 5 and 6). The handle assemblies 12 are operable to manipulate/operate the capsulorhexis forceps 10. As indicated in FIGs. 1-2A and 4A-4C, the capsulorhexis forceps 10 include a forceps tip 11. The forceps tip 11 includes an elongated tip body 15. In some implementations, the tip body 15 may extend approximately 2 to 4 inches (50 mm to 100 mm) in length. However, the tip body 15 may have a length larger or smaller than the above-indicated range.

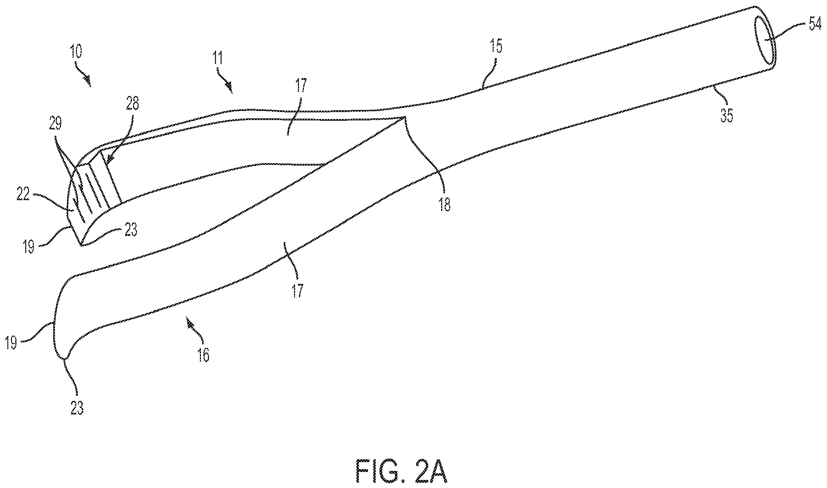

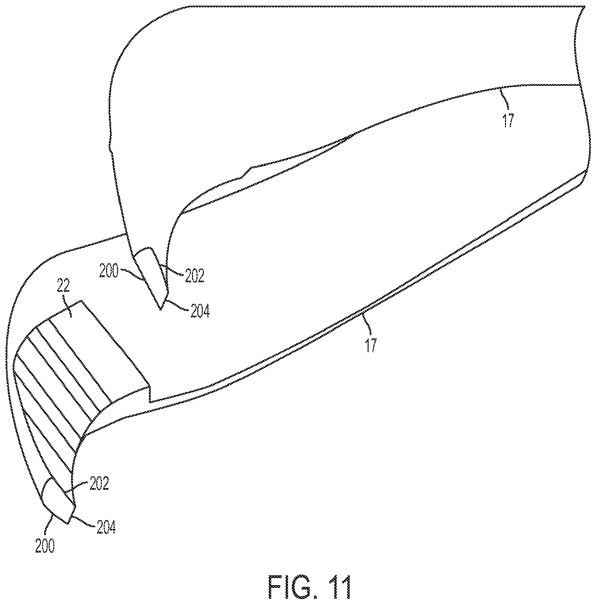

A pair of resilient pincers 17 is formed at a distal end 16 of the tip body 15. The pincers 17 extend from a location 18 along the tip body 15. Further, the pincers 17 include grasping surfaces 22 and tips 19 formed at distal ends thereof. Further, in some implementations, the tips 19 may terminate in a pointed end 23. In other implementations, the tips 19 may not include a pointed end. Rather, in some implementations, the tips 19 may be blunt In still other implementations, the tips 19 may have a hooked shape, as shown, for example, in FIG. 2A. The hook-shaped tip 19 may also include a pointed end 23. Alternately, the hook-shaped tips 19 may not include a pointed end. As shown, the pincers 17 are spaced apart from each other in a spread-apart or open configuration ("open configuration"). Referring to FIG. 11, the grasping surfaces 22 may include one or more sharp edges, such as one or more sharp edges 200, 202, 204. The sharp edges 200, 202, 204 may be used to penetrate a lens capsule of an eye. Still further, as shown in FIG. 2A, the grasping surfaces 22 may have a plurality of serrations 29 formed therein. In some implementations, the spacing between serrations may be 0.1 mm or less. However, in other implementations, the spacing between serrations may be greater than 0.1 mm.

The grasping surfaces 22 may be used to grasp the lens capsule of an eye, such as, by pinching the capsule between the grasping surfaces 22. An opening in the lens capsule may be made by tearing the capsule with the forceps while the lens capsule is pinched between the grasping surfaces 22. In other instances, a forceps tip 11 may be used to puncture the lens capsule. For example, a forceps tip 11 that includes one or more of sharp edges 200, 202, 204 may be used to puncture the lens capsule. An opening may be increased in size by grasping the lens capsule with the forceps tip 11 and tearing the capsule tissue to enlarge the opening.

The forceps tip 11 may be formed from a moldable material. For example, the forceps tip 11 may be formed from a polymeric material that is formable, such as via injection molding. Thus, the forceps tip 11 may be utilized as a disposable forceps tip. For example, the forceps tip 11 may be used as a disposable insert that can be adjustably mounted within a reusable handle 12, as indicated in Figs. 5 and 6. Thus, as a disposable insert, the forceps tip 11 may be disposed of after use. In other instances, the forceps tip 11 along with the handle 12 may be a single-use instrument that is disposable in its entirety at the conclusion of a use, such as a surgical procedure.

In some implementations, the forceps tip 11 may be molded or otherwise formed from a synthetic or composite material. For example, the forceps tip 11 may be formed from a medical grade plastic. In still other implementations, the forceps tip 11 may be formed from other materials, such as a metal injection molded material that is suitable for medical applications. For example, a polyoxymethylene acetyl copolymer high-strength medical grade resin material, or other, similar moldable copolymer material may be used. Particularly, a high strength, medical grade material that is suitable for both medical use and for microinjection molding may be used.

In addition, a medical grade moldable material, such as one or more of the materials described above, may also be enriched with other fibers or materials (referred to collectively as "additives") to enhance strength, rigidity, and/or resiliency. Example additives may include approximately 5-30% glass bead or 1-10% glass fiber. However, higher or lower concentrations of one or more of the above additives or other suitable additives may also be used to enhance physical properties of the forceps tip 11, such as, for example, to provide enhanced strength, rigidity, and/or resiliency to the forceps tip 11.

Still further, in other implementations, the forceps tip 11 also may be made from metal injection molded materials utilizing a metal injection molding process. For example, the forceps tip 11 may be formed using a material such as a stainless steel or other moldable/metal injectable materials. Particularly, metal injectable materials suitable for use in medical applications may be used to form the forceps tip 11.

As indicated above, the pincers 17 may be formed such that the pincers 17 are biased in the open configuration. The pincers 17 are moveable toward each other into a second, substantially closed or grasping/gripping position ("grasping configuration). In the grasping configuration, the pincers 17 are displaced towards each other. In the grasping configuration, the pincers 17 and particularly the tips 19 are operable to grasp objects, such as tissues, with the grasping surfaces 22. For example, in some implementations, the tips 19 of the pincers 17 may be utilized to engage and grip a capsulorhexis flap formed in the patient's eye or a replacement lens for positioning into the patient's eye

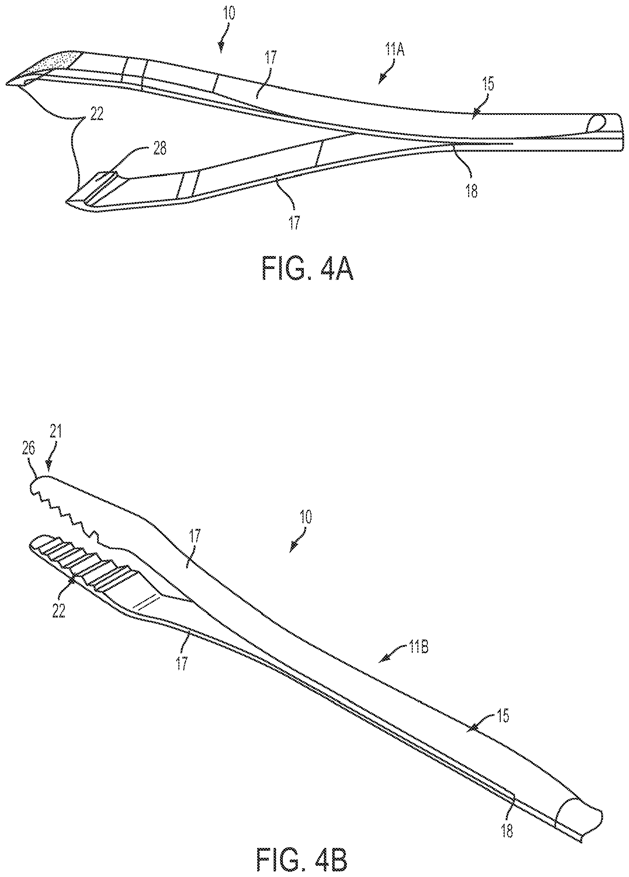

FIGs. 4A-4C illustrate other example implementations of the forceps tip 11A-11C having differing grasping surfaces 22. FIGs. 4A and 7 illustrate the forceps tip 11A in which the grasping surfaces 22 are angled. FIG. 7 shows a detail view of a distal end of a pincer 17 shown in FIG. 4A. As shown, the grasping surface 22 may be disposed at an angle θ relative to longitudinal axis 24.

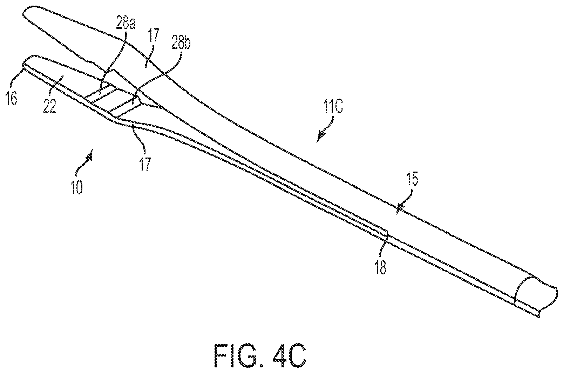

The grasping surfaces 22 may include surface structures, features, and/or texturing operable to enhance grasping and adhesion performance of the forceps 10. For example, addition of one or more of these or other features to the grasping surface 22 may provide enhance performance when utilized to grasp and retain a lens, a membrane, or a capsulorhexis flap. FIG. 4B illustrates another implementation in which the grasping surfaces 22 include a plurality of ridges, grooves, serrations, or teeth (collectively referred to as "serrations") 29. In some instances, the spacing between adjacent serrations 29 may be 0.13 mm. In other instances, the serration 29 may have any number of shapes. For example, the serrations 29 may have a pyramid, cubical, rounded, or other raised shape. Alternately, the grasping surface 22 may be substantially flat. In still other instances, the grasping surface 22 may have a desired surface roughness. Further, as shown in FIGS. 2A, 4A, 4B, and 4C, the grasping surfaces 22 may be formed on a raised shelf 28. Also, as shown in FIG. 4C, the raised shelf may be a plurality of raised shelves offset from each other, such as shelves 28a and 28b. The plurality of shelves 28a and 28b results in the grasping surface 22 form a tiered surface. In still other implementations, the grasping surfaces 22 may include blind holes, recesses, or other surface features to enhance grasping and adhesion properties of the forceps 10.

Referring again to FIGs. 1 and 2A, a proximal end 35 of the tip body 15 generally is positioned proximally of the intermediate point 18 and pincers 17. The proximal end 35 defines a connection end of the forceps tip 11. The second end 35 is operable to connect the forceps tip 11 to a handle, such as the example handle 12 shown in FIGs. 5 and 6.

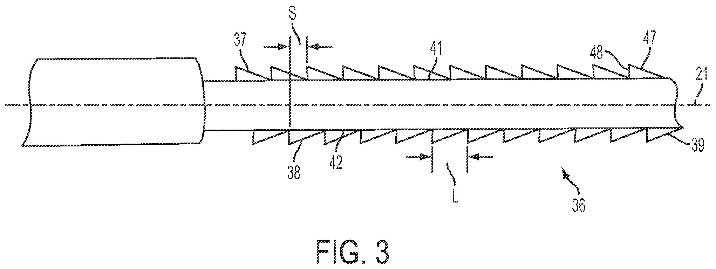

In some implementations, the second end 35 may include a tip connector 36. Example tip connectors 36 are illustrated in FIGs. 1, 2B, and 3. The tip connector 36 may include a first set of teeth 37 and a second set of teeth 38. Referring to FIG. 3, the first set of teeth 37 is formed along a first side 41 of the tip body 15 adjacent the proximal end 35, and the second set of teeth 38 is formed along a second side 42 of tip body 15, opposite the first side 41, adjacent the proximal end 35. Each of the first set of teeth 37 and the second set of teeth 38 may include a plurality of teeth 39. The teeth 39 may have a first surface 47 and a second surface 48. As shown in FIG. 3, the first surface 47 slopes outwardly (i.e., away from the longitudinal axis 21) towards the distal end 16. The second surface 48 may be perpendicular or substantially perpendicular to the longitudinal axis 1.

Each tooth 39 of the first and second sets of teeth 37, 38 may include a tooth length L defined as the distance between a location where adjacent second surfaces 48 meet the tip body 15. In some instances, the tooth length L may be approximately 0.1 mm to 0.15 mm. In other instances, the tooth length L may vary from this range. For example, in some instances, the tooth length L may be greater than 0.15 mm or less than 0.1 mm. The first set of teeth 37 may be longitudinally offset from the second set of teeth 38 about longitudinal axis 21. That is, in some implementations, teeth 39 within the first set of teeth 37 are staggered from and not aligned with teeth 39 within the second set of teeth 38. As shown in FIG. 3, the teeth 39 within the first set of teeth 37 are offset from the teeth 39 in the second set of teeth 38 by a distance S. In some implementations, the distance S may be one half of the tooth length L. In some implementations, the distance S may be within the range of approximately 0.05 mm to approximately 0.075 mm. However, the distance S may be any desired distance. For example, a larger or smaller distance S may be used. Further, the distance S may vary depending on, for example, the tooth length L or may be independent of tooth length L.

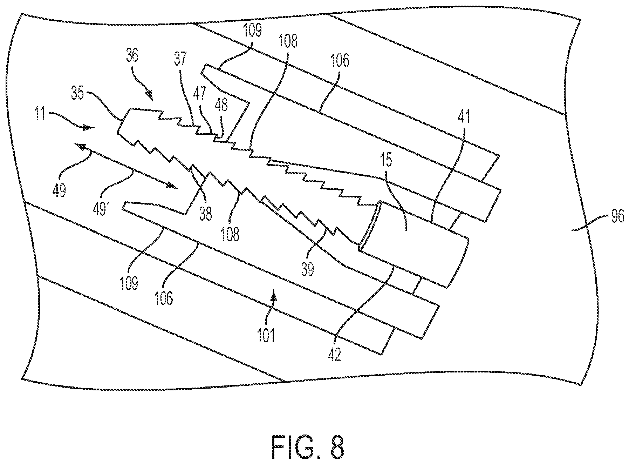

Referring to FIG. 3, the staggered relationship between the first set of teeth 37 and the second set of teeth 38 may be utilized to provide a stepped adjustment of the position of the forceps tip 15 within the handle 12 by an amount that is smaller than the tooth length L. Thus, in some implementations, a position of the forceps tip 11 relative to the handle 12 may be adjusted incrementally by successive distances S, for example, in the directions of arrow 49, as shown, for example, in FIG. 8. It further will be understood that other spacings or arrangements of the first set of teeth 37 and the second set of teeth 38, as well as the use of fewer or additional sets of teeth, may also be provided as needed or desired.

The teeth 39 may be integrally formed with the tip body 15. For example, the teeth 39 may be molded, extruded, or otherwise formed with the tip body 15. In other implementations, the teeth 39 may be formed separately from tip body 15. For example, the tip connector 36 including the first and second sets of teeth 37, 38 may be formed as a separate piece and coupled to the tip body 15. For example, the tip connector 36 may be coupled directly or indirectly to the tip body. Further, the tip connector 36 may be coupled to the tip body by, for example, welding, an adhesive, press fit, interlocking mechanism, or in any other suitable way.

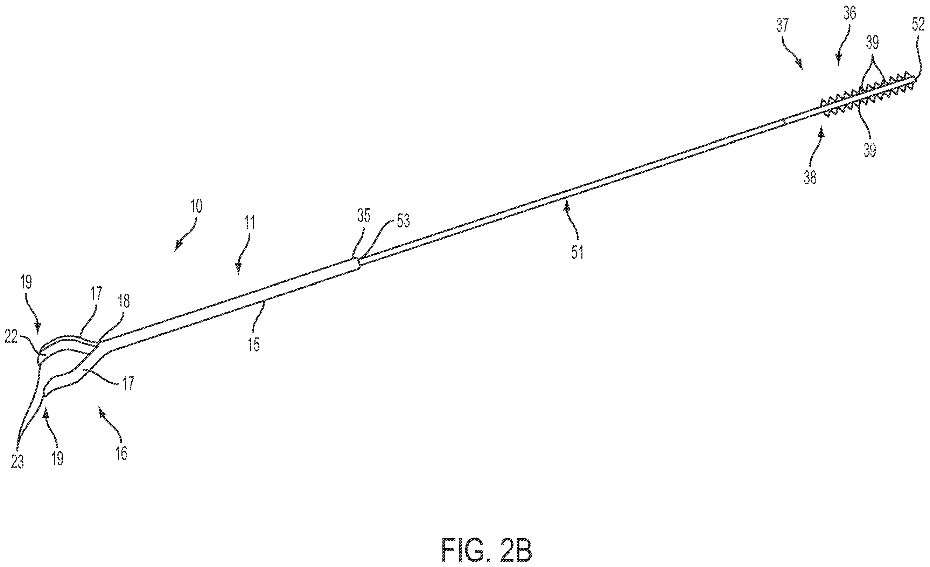

FIGs. 2A and 2B illustrate another example implementation of the forceps tip 11. In this implementation, the forceps tip 11 includes a tip body 15 that is coupled to a tip extension 51. The tip body 15 may have a configuration similar to that explained above and may include a receptacle 54. In some instances, the tip extension 51 may be an elongated wire or rod. A distal end 53 of the extension 51 is received into the receptacle 54 of the tip body 15. A proximal end 52 of the extension 51 may include a tip connector 36. The tip connector 36 may be configured as explained above. A length of each of the tip body 15 and the extension 51 may be any desired length. In some instances, the tip body 15 may have a length of approximately one half of an inch to one inch (i.e., 12.5 mm to 25 mm)

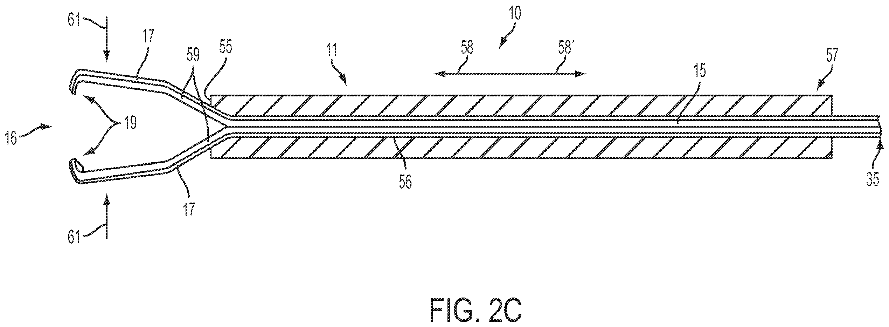

Referring to FIG. 2C, a sleeve 57 may be received over at least a portion of the tip body 15 of the forceps tip 11. For implementations utilizing a tip extension 51, the sleeve 57 may be received over a portion of the tip extension 51. The sleeve 57 is slideable over the forceps tip 11 in the direction arrows 58 and 58'. As the sleeve. 57 is moved along the tip body 11 in the direction of arrow 58, a distal end 55 engages proximal portions 59 of the pincers 17, causing the tips 19 to be urged inwardly toward each other in the direction of arrows 61. As the pincers 17 are displaced towards each other, the proximal portions 59 of the pincers 17 are at least partially received into passage 56 defined by the sleeve 57. Consequently, movement of the sleeve 57 in the direction of arrow 58 is operable to move the pincers 17 into a closed or grasping configuration. Movement of the sleeve 57 in the direction of arrow 58' causes the proximal portions 59 to be removed from the passage 56. As a result, the tips 19 of the pincers 17 move away from each other due to the resilient quality of the pincers 17. Therefore, the displacement of the sleeve 57 in the directions of 58 and 58' is operable to close and open the pincers 17, respectively. In the context of a capsulorhexis procedure, the interaction between the sleeve 57 and the pincers 17 is operable to cause the pincers 17, for example, to grasp a capsulorhexis flap or to grip a replacement lens for insertion into a patients eye.

The sleeve 57 may be formed from any suitable material. For example, the sleeve 57 may be formed from a metal or polymeric material. Particularly, the sleeve 57 may be formed from materials that are medical grade quality. Further, the material selected to form the different components described herein may be selected based, at least in part, on whether the component is to be part of a reusable instrument or a single use disposable instrument.

FIGs. 5 and 6 illustrate different example devices 100, 200. The implementations, of the handle 12 that can be used with the forceps tip 11. The handle 12 may be formed from a variety of materials. For example, in some instances, the handle 12 may be formed from one or more plastics, synthetic materials (e.g., synthetic fibers or synthetic diamond), composites, and/or metals. Further, as explained above, a material selected for forming the handle 12 may be based, at least in part, on whether the device, e.g., devices 100, 200, is to be re-useable or a disposable.

The handle 12 may include arm elements 73 and 74. As shown in FIGS. 5 and 6, an elongate element 96 is disposed between the arm elements 73, 74. The arm elements 73 and 74 are coupled at a union 79 at a proximal end 72 of the handle 12 and diverge outwardly away from the elongate element 96. Referring to the example device 200 of FIG. 6, a proximal end 79 of the elongate member 96 may include one or more mating features 98 (e.g., protrusions, projections, teeth, roughened surface, ribs, etc.) formed on an outer surface of the elongate member 96 that engage with corresponding engaging features 99 formed on an inner surface 94 of the handle 12. The engaging features 98, 99 cooperate to lock the elongate member 96 in place.

The devices 100, 200 may also include a tip guide 85. The tip guide 85 may include a sleeve 57 and a hub 86. In some instances, the sleeve 57 and the hub 86 may be formed from the same material. Further, in some instances, the sleeve 57 and the hub 86 may be integrally formed. In other instances, the sleeve 57 and the hub 86 may be formed from different materials. For example, in some instances, the sleeve 57 may be formed from a metal, such as stainless steel, whereas the hub 86 may be formed from a polymeric material. Additionally, the hub 86 may have a semi-hemispherical shape (as shown in FIG. 6) or a cylindrical shape (as shown in FIG. 5). However, the hub 86 may have any desired shape.

Referring to FIG. 6, the tip guide 85 may be coupled to the arm elements 73, 74 via resilient elements 88. The resilient elements 88 may be coupled to the arm elements 73, 74 at distal ends 71 thereof. As explained in more detail below, the resilient elements 88 provide a biasing formed to return the pincers of the forceps tip to an open configuration. In some implementations, the arm elements 73, 74; the resilient elements 88; and the hub 86 may be integrally formed. Referring to FIG. 5, the device 100 includes four hinged elements 89 that are connected to the arm elements 73, 74 and the tip guide 85 via hinges 102 and 104. Additional or fewer hinged elements 89 may be included. The hinged elements 89 are operable to pivot about hinges 102 and 104 to displace the sleeve 57 distally when the arm elements 73, 74 are urged towards each other. Operation of the devices 100, 200 is described in more detail below.

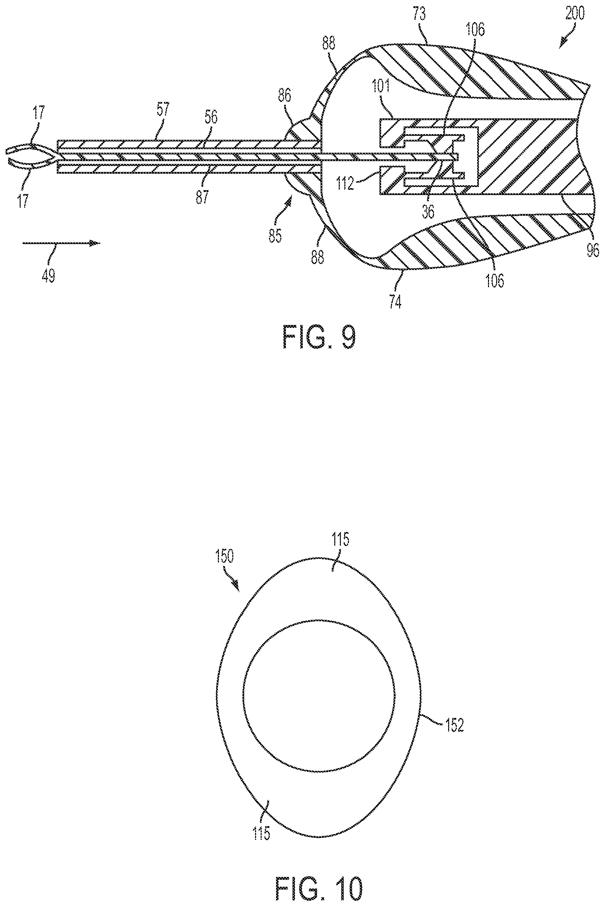

As shown in FIG. 9, the tip guide 85 defines a passage 87. A forceps tip 11 extends through the passage 87. As explained above, the forceps tip 11 may include a tip connector 36. The tip connector 36 is adapted to couple to the device 200 via a locking mechanism 101. The locking mechanism 101 may be formed in a distal end 112 of the elongate element 96. Although the locking mechanism 101 is described in relation to the device 200, the device 100 may also include a locking mechanism that is similar to the locking mechanism 101.

A detail view of the locking mechanism 101 is shown in FIG. 9. The locking mechanism 101 is operable to adjustably secure the forceps tip 11. The locking mechanism 101 includes locking members 106. In some instances, the locking members 106 include mating teeth 108 that cooperatively engage with the teeth 39 of the tip connector 36 as illustrated in FIG. 8. As shown in FIG. 8, the mating teeth 108 are adapted to engage the first and second set of teeth 37, 38 formed on the tip connector 36. As the forceps tip 11 is moved in the direction of arrow 49, the teeth 39 of the first and second sets of teeth 37, 38 are passed between the locking members 106. Particularly, as the teeth 39 move between the locking members 106, the sloping surface of the first surface 47 of the teeth 39 causes the locking members 106 to be deflected outwardly away from the forceps tip 11. As one of the teeth 39 extends past the ends of the locking members 106, the locking members 106 return to their initial positions.

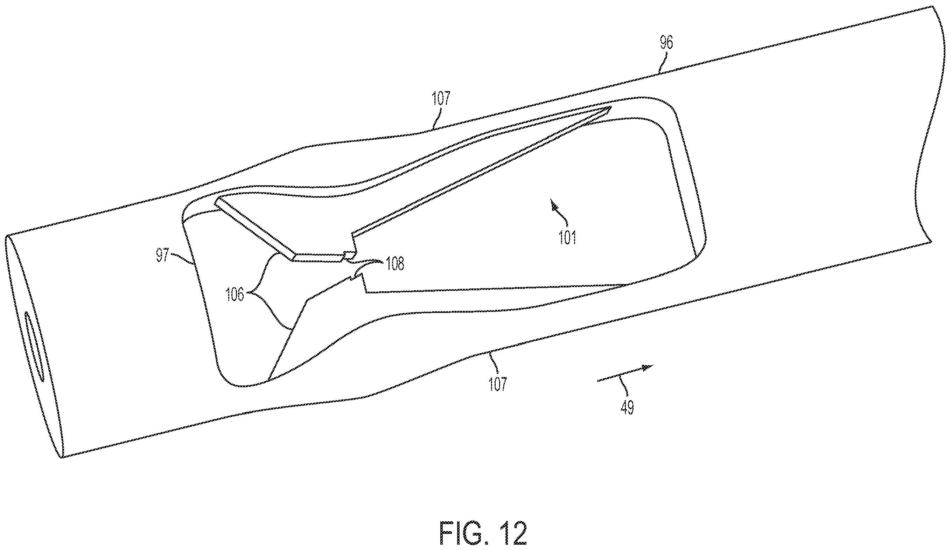

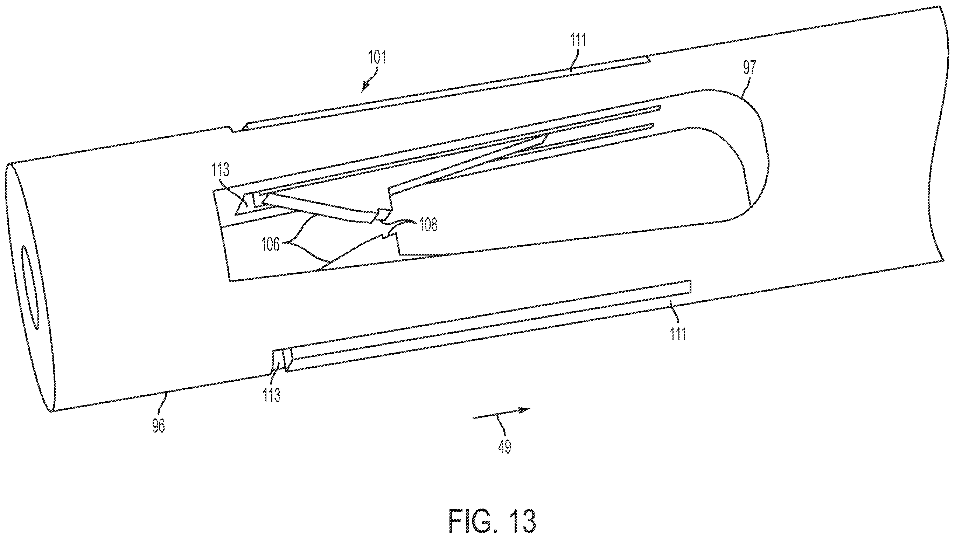

FIGS. 12 and 13 illustrate alternative locking mechanisms 101. Referring to FIG. 12, the elongate member 96 defines a slot 97. The locking mechanism 101 includes locking members 106 disposed in the slot 97. The locking members 106 are integrally formed with walls 107 defining the slot 97 and include mating teeth 108. Similar to those described above, the mating teeth 108 are adapted to cooperatively engage with teeth 39 formed on the forceps tip 11. As the forceps tip 11 (not shown) is passed between the locking members 106, the teeth 39 of the forceps tip 11 are operable to pass in the direction of arrow 49 past the mating teeth 108. As the teeth 39 pass the mating teeth 108, the teeth 39 cause the locking member 106 to separate laterally. Thus, the walls 107 flex in response to movement of the forceps tip 11 between the locking members 106. Also similar to the locking mechanism 101 shown in FIG. 8, the locking members 106 prevent movement of the forceps tip 11 in the direction opposite of arrow 49 due to the interlocking fit between teeth 39 and mating teeth 108.

Referring to FIG. 13, again, the elongate member 96 defines a slot 97. The locking members 106 protrude into the slot 97 and are adapted to engage the teeth 39 of the forceps tip 11 with mating teeth 108. The locking members 106 are pivotably coupled to the elongate member 96 at bases 111. Each of the locking members 106 is disposed in a slot 113. The slots 113 may be formed perpendicular to the slot 97. Further, in some implementations, the locking members 106 may be formed integrally with the elongate member 96. The locking members 106 are adapted to flex laterally outwards as the teeth 39 of the forceps tip 11 pass the mating teeth 108 when the forceps tip 11 is moved in the direction of arrow 49. Similar to the locking members 106 of the locking mechanism 101 shown in FIGs. 8 and 12, the locking members 106 are biased to return to their original position in response to movement of the teeth 39 and mating teeth 108 relative to each other. Also, interlocking of the teeth 39 and mating teeth 108 prevents movement of the forceps tip 11 relative to the elongate member 96 in the direction opposite arrow 49.

In their initial positions, the second surface 48 of the teeth 39 (shown in FIG. 3) lockingly engage corresponding surfaces of the teeth 108, preventing movement of the forceps tip 11 in the direction of 49'. Consequently, the locking mechanism 101 is adapted to permit movement of the forceps tip 11 in only one direction, i.e., direction 49. As indicated above, in some implementations, the teeth 39 of the first and second sets of teeth 37, 38 may be offset from each other by a distance S. The teeth 108 may similarly be offset by a distance S. Thus, in some implementations, as one tooth 39 of either the first set of teeth 37 or the second set of teeth 38 move past one of the locking members 106 adjacent thereto, the interaction between one of the teeth 39 with the teeth 108 is operable to immobilize the forceps tip 11 within the device 200. Further, the offset distance S between the first and second sets of teeth 37, 38 allows for stepped adjustment in position of the forceps tip 11, providing a more precise location of the forceps tip 11 within the device 200. This is particularly important in order to precisely locate the pincers 17 relative to the distal end 55 of the sleeve 57. Precisely locating the pincers 17 relative to the sleeve 57 allows, for example, the pincers to be in their fully open configuration when the device 100, 200 is in an unactuated condition and immediate movement of the pincers 17 towards each other when a user applies a force to urge the arm elements 73, is toward each other due to contact of the distal end 55 of the sleeve 57 with the pincers 17. In some instances, positioning of the forceps tip 11 within the locking mechanism 101 may be performed during assembly and fixed in place with an adhesive, for example. However, in other instances, the forceps tip 11 may be positioned within the locking mechanism 101 at other times.

The locking members 106 may also include protrusions 109. The protrusions 109 may be moved away from each other to release the forceps tip 11 from engagement with the locking members 106. This may be useful if the forceps tip 11 has been positioned too far in the direction of arrow 49. Separation of the protrusions 109 and, hence, the locking members 106 allows for the forceps tip 11 to be repositioned. For example, the forceps tip 11 may be moved in the position of arrow 49' upon separation of the protrusions 109.

Referring to FIGs. 2C and 6, operation of the device 200 is described. However, the described operation is also applicable to the device 100. In an at-rest condition, the pincers 17 of the device 200 are in a fully-open state. When the arm elements 73, 74 are urged towards each other, the sleeve 57 and hub 86 are displaced in the direction of arrow 58 via distal movement of ends of the resilient elements 88 that are coupled to the hub 86. The distal end 55 of the sleeve 57 engages the pincers 17, causing the tips 19 of the pincers 17 to move towards each other into a closed configuration. As the arm elements 73, 74 are moved closer together, the tips 19 are, accordingly, moved closer together. As a force applied to the arm elements 73, 74 is removed the arm elements 73, 74 return to their at-rest position. In response the resilient elements 88 displace sleeve 57 and hub 86 in the direction of arrow 58' relative to the forceps tip 11, causing the tips 19 to move away from each other due to the resilient properties of the pincer 17. The pincers 17 continue to move away from each other until the arm elements 73, 74 have attained their at-rest position.

Referring to FIGs. 6 and 10, the device 200 may include a sleeve 150. The sleeve 150 may be positioned over the interface between the locking mechanism 101 and the tip connector 36 of the forceps tip 11. Additionally, the sleeve 150 may include a cam-shaped lip 152. The cam shape of the lip 152 defines lobes 115. The lobes 115 may be positioned relative to one of the arm elements 73, 74 by rotation of the sleeve 150 about longitudinal axis 114. The rim 152 and, particularly, the lobes 115 limit an amount of actuation of the device 200 as a result of contact between the arm elements 73, 74 and the rim 152. The amount of displacement of the arm elements 73, 74 may be varied based on the rotational position of the rim 152 relative to the arm elements 73, 74. A position of the lobes 115 relative to the arm elements 73, 74 defines an endstop to limit an amount of displacement of the arm elements 73, 74. Thus, a position where one of the arm elements 73, 74 contacts rim 152 defines a fully actuated position of the device 200. Consequently, a rotational position of the rim 152 is operable to limit an amount by which the sleeve 57 is moved relative to the forceps tip 11. A position of the sleeve 150 may be selected during assembly of the device 200 or some other time. Further, the sleeve 150 may be fixed into place, for example, with an adhesive. While an example sleeve 150 is shown with a pair of lobes 115, other sleeves 150 may have a different number of lobes 115. For example, another example sleeve 150 may have a single lobe 115. In other instances, the sleeve 150 may have more than two lobes 115.

Accordingly, it can be seen that the present disclosure provides for a forceps tip and, more generally, a forceps device that can be economically and efficiently mass produced from molded medical grade materials. The forceps device may be utilized in a capsulorhexis procedure. Further, the forceps device may be disposable after a single use. The forceps tip may also be easily and securely inserted into and removed from a handle of the forceps device, with the position of the forceps tip being adjustable with respect to the handle.

The foregoing description generally illustrates and describes various implementations of the present disclosure. It will, however, be understood by those skilled in the art that various changes and modifications can be made to the above-discussed description without departing from the scope of the disclosure. It is intended that all matter contained in the above description or shown in the accompanying drawings shall be interpreted as being illustrative, and not to be taken in a limiting sense. Furthermore the scope of the present disclosure shall be construed to cover various modifications, combinations, additions, alterations, etc. above and to the above-described embodiments, which shall be considered to be within the scope of the present disclosure. Accordingly, various features and characteristics of the present disclosure as discussed herein may be selectively interchanged and applied to other illustrated and non-illustrated implementations, and numerous variations, modifications, and additions further can be made thereto without departing from the scope of the present disclosure as set forth in the appended claims.

## Claims

1. A forceps device, comprising:
a handle (12) comprising:
a first arm element (73);
a second arm element (74), the first arm element (73) and the second arm element (74) joined at a first end of the handle (12);
an elongate body (96) extending between the first arm element (73) and the second arm element (74); and
a locking mechanism (101) formed in the elongate body (96), the locking mechanism (101) comprising a first locking member (106) and a second locking member (106), the first locking member (106) and the second locking member (106) being laterally movable with respect to each other and with respect to a longitudinal axis (114) of the locking mechanism (101);
a forceps tip (11) coupled to the handle (12), the forceps tip (11) comprising:
a pair of pincers (17) extending from a distal end of the forceps tip (11), the pair of pincers (17) laterally offset from each other to define an open configuration and moveable between the open configuration and a closed configuration in which the pair of pincers (17) contact each other; and
a tip connector (36) extending from a proximal end of the forceps tip (11), the tip connector (36) adapted to interlock with the locking mechanism (101); and
a sleeve (57) coupled to the arm elements and defining a central passage, the forceps tip (11) extending through the central passage, the sleeve (57) operable to move relative to the forceps tip (11) and actuate the pincers (17) between the open configuration and the closed configuration in response to lateral displacement of the arm elements (73, 74); **characterized in that** :
the tip connector (36) interlocks with the locking mechanism (101) such that when interlocked with the locking mechanism (101), the tip connector (36) is movable in a first axial direction (49) relative to the locking mechanism (101) and is prevented from moving in a second axial direction relative to the locking mechanism (101), the second axial direction being opposite the first axial direction.

2. The forceps device of claim 1, further comprising resilient elements (88) extending between distal ends of the arm elements and the sleeve (57).

3. The forceps device of claim 1, wherein a distal end of at least one of the pincers (17) comprises a grasping surface (22).

4. The forceps device of claim 3, wherein the grasping surface comprises a series of serrations (29).

5. The forceps device of claim 1, wherein the forceps tip (11) comprises a tip body (15), the tip body (15) including the pair of pincers (17), and a tip extension coupled to the tip body (15), the tip extension including the tip connector (36).

6. The forceps device of claim 1, wherein the tip connector (36) comprises a first plurality of teeth (37, 38) arranged longitudinally along the forceps tip (11).

7. The forceps device of claim 6, wherein the locking mechanism (101) comprises a second plurality of teeth (108), wherein the second plurality of teeth (108) are configured to interlock with the first plurality of teeth (37, 38) to couple the forceps tip (11) to the handle (12).

8. The forceps device of claim 6, wherein the first plurality of teeth (37, 38) comprise:
a first set of teeth (37) extending longitudinally along a first side (41) of the tip connector (36); and
a second set of teeth (38) extending longitudinally along a second side (42) of the tip connector (36).

9. The forceps device of claims 7 and 8, wherein the second plurality of teeth (108) comprises:
a third set of teeth configured to interlock with the first set of teeth (37); and
a fourth set of teeth configured to interlock with the second set of teeth (38).

10. The forceps device of claim 8, wherein the first set of teeth (37) and the second set of teeth (38) are longitudinally offset from each other.

11. The forceps device of claims 9 and 10, wherein the third set of teeth and the fourth set of teeth are offset from each other by the same amount the first set of teeth (37) is offset from the second set of teeth (38).

12. The forceps device of claim 1, wherein the forceps tip (11) is formed from a plastic injection molded material.

13. The forceps device of claim 1, wherein the forceps tip (11) is formed from a metal injection molded material.

14. The forceps device of claim 1, wherein the locking mechanism (101) further comprises a third set of teeth formed on the first locking member (106) and a fourth set of teeth formed on the second locking member (106).

15. The forceps tip (11) of claim 1, wherein the handle (12) further comprises:
a first resilient element (88) extending between the first arm element (73) and the sleeve (57); and
a second resilient element (88) extending between the second arm element (74) and the sleeve (57).

## Patentansprüche

1. Pinzettenvorrichtung, umfassend:
einen Griff (12), umfassend:
ein erstes Armelement (73);
ein zweites Armelement (74), wobei das erste Armelement (73) und das zweite Armelement (74) an einem ersten Ende des Griffs (12) miteinander verbunden sind;
einen langgestreckten Körper (96), der sich zwischen dem ersten Armelement (73) und dem zweiten Armelement (74) erstreckt; und
einen Verriegelungsmechanismus (101), der im langgestreckten Körper (96) ausgebildet ist, wobei der Verriegelungsmechanismus (101) ein erstes Verriegelungselement (106) und ein zweites Verriegelungselement (106) umfasst, wobei das erste Verriegelungselement (106) und das zweite Verriegelungselement (106) bezüglich einander und bezüglich einer Längsachse (114) des Verriegelungsmechanismus (101) seitlich bewegbar sind;
eine Pinzettenspitze (11), die mit dem Griff (12) verbunden ist, wobei die Pinzettenspitze (11) Folgendes umfasst:
ein Paar Greifer (17), die sich von einem distalen Ende der Pinzettenspitze (11) erstrecken, wobei das Paar Greifer (17) seitlich voneinander versetzt ist, um eine offene Konfiguration zu definieren, und zwischen der offenen Konfiguration und einer geschlossenen Konfiguration, in der das Paar Greifer (17) einander berühren, bewegbar ist; und
einen Spitzenverbinder (36), der sich von einem proximalen Ende der Pinzettenspitze (11) erstreckt, wobei der Spitzenverbinder (36) zur Verriegelung mit dem Verriegelungsmechanismus (101) ausgelegt ist; und
eine Hülse (57), die mit den Armelementen verbunden ist und einen zentralen Durchgang definiert, wobei sich die Pinzettenspitze (11) durch den zentralen Durchgang erstreckt, wobei die Hülse (57) betätigt werden kann, um sich bezüglich der Pinzettenspitze (11) zu bewegen und die Greifer (17) als Reaktion auf eine seitliche Verschiebung der Armelemente (73, 74) zwischen der offenen Konfiguration und der geschlossenen Konfiguration zu bewegen; **dadurch gekennzeichnet, dass**:
der Spitzenverbinder (36) mit dem Verriegelungsmechanismus (101) verriegelt wird, derart, dass der Spitzenverbinder (36), wenn er mit dem Verriegelungsmechanismus (101) verriegelt ist, in eine erste axiale Richtung (49) bezüglich des Verriegelungsmechanismus (101) bewegbar ist, und an einer Bewegung in eine zweite axiale Richtung bezüglich des Verriegelungsmechanismus (101) gehindert wird, wobei die zweite axiale Richtung zur ersten axialen Richtung entgegengesetzt ist.

2. Pinzettenvorrichtung nach Anspruch 1, ferner umfassend federnd nachgiebige Elemente (88), die sich zwischen distalen Enden der Armelemente und der Hülse (57) erstrecken.

3. Pinzettenvorrichtung nach Anspruch 1, wobei ein distales Ende mindestens eines der Greifer (17) eine Greiffläche (22) umfasst.

4. Pinzettenvorrichtung nach Anspruch 3, wobei die Greiffläche eine Reihe von Verzahnungen (29) umfasst.

5. Pinzettenvorrichtung nach Anspruch 1, wobei die Pinzettenspitze (11) einen Spitzenkörper (15) umfasst, wobei der Spitzenkörper (15) das Paar Greifer (17) aufweist, und eine mit dem Spitzenkörper (15) verbundene Spitzenverlängerung, wobei die Spitzenverlängerung den Spitzenverbinder (36) aufweist.

6. Pinzettenvorrichtung nach Anspruch 1, wobei der Spitzenverbinder (36) eine erste Vielzahl von Zähnen (37, 38) umfasst, die längs entlang der Pinzettenspitze (11) angeordnet sind.

7. Pinzettenvorrichtung nach Anspruch 6, wobei der Verriegelungsmechanismus (101) eine zweite Vielzahl von Zähnen (108) umfasst, wobei die zweite Vielzahl von Zähnen (108) zur Verriegelung mit der ersten Vielzahl von Zähnen (37, 38) zur Verbindung der Pinzettenspitze (11) mit dem Griff (12) ausgelegt ist.

8. Pinzettenvorrichtung nach Anspruch 6, wobei die erste Vielzahl von Zähnen (37, 38) Folgendes umfasst:
einen ersten Satz von Zähnen (37), die sich längs entlang einer ersten Seite (41) des Spitzenverbinders (36) erstrecken; und
einen zweiten Satz von Zähnen (38), die sich längs entlang einer zweiten Seite (42) des Spitzenverbinders (36) erstrecken.

9. Pinzettenvorrichtung nach Anspruch 7 und 8, wobei die zweite Vielzahl von Zähnen (108) Folgendes umfasst:
einen dritten Satz von Zähnen, der zur Verriegelung mit dem ersten Satz von Zähnen (37) ausgelegt ist; und
einen vierten Satz von Zähnen, der zur Verriegelung mit dem zweiten Satz von Zähnen (38) ausgelegt ist.

10. Pinzettenvorrichtung nach Anspruch 8, wobei der erste Satz von Zähnen (37) und der zweite Satz von Zähnen (38) in Längsrichtung voneinander versetzt sind.

11. Pinzettenvorrichtung nach Anspruch 9 und 10, wobei der dritte Satz von Zähnen und der vierte Satz von Zähnen um denselben Betrag voneinander versetzt sind wie der erste Satz von Zähnen (37) vom zweiten Satz von Zähnen (38) versetzt ist.

12. Pinzettenvorrichtung nach Anspruch 1, wobei die Pinzettenspitze (11) aus einem spritzgegossenen Kunststoffmaterial geformt ist.

13. Pinzettenvorrichtung nach Anspruch 1, wobei die Pinzettenspitze (11) aus einem spritzgegossenen Metallmaterial geformt ist.

14. Pinzettenvorrichtung nach Anspruch 1, wobei der Verriegelungsmechanismus (101) ferner einen dritten Satz von Zähnen, der auf dem ersten Verriegelungselement (106) ausgebildet ist, und einen vierten Satz von Zähnen, der auf dem zweiten Verriegelungselement (106) ausgebildet ist, umfasst.

15. Pinzettenspitze (11) nach Anspruch 1, wobei der Griff (12) ferner Folgendes umfasst:
ein erstes federnd nachgiebiges Element (88), das sich zwischen dem ersten Armelement (73) und der Hülse (57) erstreckt; und
ein zweites federnd nachgiebiges Element (88), das sich zwischen dem zweiten Armelement (74) und der Hülse (57) erstreckt.

## Revendications

1. Dispositif de pincette comprenant :
un manche (12) comprenant :
un premier élément formant branche (73) ;
un second élément formant branche (74), le premier élément formant branche (73) et le second élément formant branche (74) étant raccordés au niveau d'une première extrémité du manche (12) ;
un corps allongé (96) s'étendant entre le premier élément formant branche (73) et le second élément formant branche (74) ; et
un mécanisme de verrouillage (101) formé dans le corps allongé (96), le mécanisme de verrouillage (101) comprenant un premier organe de verrouillage (106) et un second organe de verrouillage (106), le premier organe de verrouillage (106) et le second organe de verrouillage (106) pouvant être déplacés latéralement l'un par rapport à l'autre et par rapport à un axe longitudinal (114) du mécanisme de verrouillage (101) ;
un embout formant pincette (11) accouplé au manche (12), l'embout formant pincette (11) comprenant :
une paire de mâchoires (17) s'étendant à partir d'une extrémité distale de l'embout formant pincette (11), la paire de mâchoires (17) étant décalées latéralement l'une de l'autre de façon à définir une configuration ouverte et pouvant être déplacées entre la configuration ouverte et une configuration fermée, dans laquelle la paire de mâchoires (17) se trouvent en contact l'une avec l'autre ; et
un organe de raccordement d'embout (36) s'étendant à partir d'une extrémité proximale de l'embout formant pincette (11), l'organe de raccordement d'embout (36) étant conçu pour venir en prise avec le mécanisme de verrouillage (101) ; et
un manchon (57) accouplé aux éléments formant branches et définissant un passage central, l'embout formant pincette (11) s'étendant à travers le passage central, le manchon (57) pouvant être mis en oeuvre pour se déplacer par rapport à l'embout formant pincette (11) et actionner les mâchoires (17) entre la configuration ouverte et la configuration fermée en réaction à un déplacement latéral des éléments formant branches (73, 74) ; **caractérisé en ce que** :
l'organe de raccordement d'embout (36) vient en prise avec le mécanisme de verrouillage (101) de telle sorte que, une fois en prise avec le mécanisme de verrouillage (101), l'organe de raccordement d'embout (36) puisse être déplacé dans une première direction axiale (49) par rapport au mécanisme de verrouillage (101) et soit dans l'incapacité de se déplacer dans une seconde direction axiale par rapport au mécanisme de verrouillage (101), la seconde direction axiale étant opposée à la première direction axiale.

2. Dispositif de pincette selon la revendication 1, comprenant en outre des éléments élastiques (88) s'étendant entre des extrémités distales des éléments formant branches et le manchon (57).

3. Dispositif de pincette selon la revendication 1, dans lequel une extrémité distale d'au moins une des mâchoires (17) comprend une surface de saisie (22).

4. Dispositif de pincette selon la revendication 3, dans lequel la surface de saisie comprend une série de cannelures (29).

5. Dispositif de pincette selon la revendication 1, dans lequel l'embout formant pincette (11) comprend un corps d'embout (15), le corps d'embout (15) comprenant la paire de mâchoires (17), et un prolongement d'embout accouplé au corps d'embout (15), le prolongement d'embout comprenant l'organe de raccordement d'embout (36).

6. Dispositif de pincette selon la revendication 1, dans lequel l'organe de raccordement d'embout (36) comprend une première pluralité de dents (37, 38) disposée longitudinalement le long de l'embout formant pincette (11).

7. Dispositif de pincette selon la revendication 6, dans lequel le mécanisme de verrouillage (101) comprend une seconde pluralité de dents (108), dans lequel la seconde pluralité de dents (108) est configurée pour venir en prise avec la première pluralité de dents (37, 38) de façon à accoupler l'embout formant pincette (11) au manche (12).

8. Dispositif de pincette selon la revendication 6, dans lequel la première pluralité de dents (37, 38) comprend :
un premier ensemble de dents (37) s'étendant longitudinalement le long d'un premier côté (41) de l'organe de raccordement d'embout (36) ; et
un deuxième ensemble de dents (38) s'étendant longitudinalement le long d'un second côté (42) de l'organe de raccordement d'embout (36).

9. Dispositif de pincette selon les revendications 7 et 8, dans lequel la seconde pluralité de dents (108) comprend :
un troisième ensemble de dents configuré pour venir en prise avec le premier ensemble de dents (37) ; et
un quatrième ensemble de dents configuré pour venir en prise avec le deuxième ensemble de dents (38).

10. Dispositif de pincette selon la revendication 8, dans lequel le premier ensemble de dents (37) et le deuxième ensemble de dents (38) sont décalés longitudinalement l'un de l'autre.

11. Dispositif de pincette selon les revendications 9 et 10, dans lequel le troisième ensemble de dents et le quatrième ensemble de dents sont décalés l'un de l'autre dans la même mesure que le premier ensemble de dents (37) est décalé du deuxième ensemble de dents (38).

12. Dispositif de pincette selon la revendication 1, dans lequel l'embout formant pincette (11) est réalisé à partir d'une matière plastique moulée par injection.

13. Dispositif de pincette selon la revendication 1, dans lequel l'embout formant pincette (11) est réalisé à partir d'un matériau métallique moulé par injection.

14. Dispositif de pincette selon la revendication 1, dans lequel le mécanisme de verrouillage (101) comprend en outre un troisième ensemble de dents formé sur le premier organe de verrouillage (106) et un quatrième ensemble de dents formé sur le second organe de verrouillage (106).

15. Embout formant pincette (11) selon la revendication 1, dans lequel le manche (12) comprend en outre :
un premier élément élastique (88) s'étendant entre le premier élément formant branche (73) et le manchon (57) ; et
un second élément élastique (88) s'étendant entre le second élément formant branche (74) et le manchon (57).
